(19) Europäisches Patentamt
European Patent Office
Office européen des brevets

(11) Veröffentlichungsnummer : **0 082 542**
**B1**

(12) # EUROPÄISCHE PATENTSCHRIFT

(45) Veröffentlichungstag der Patentschrift : **15.05.85**

(51) Int. Cl.⁴ : **C 07 D213/807**

(21) Anmeldenummer : **82201240.7**

(22) Anmeldetag : **06.10.82**

(54) **Verfahren zur Herstellung von Chinolinsäure.**

(30) Priorität : **17.12.81 DE 3150005**

(43) Veröffentlichungstag der Anmeldung : **29.06.83 Patentblatt 83/26**

(45) Bekanntmachung des Hinweises auf die Patenterteilung : **15.05.85 Patentblatt 85/20**

(84) Benannte Vertragsstaaten : **CH DE FR GB LI NL**

(56) Entgegenhaltungen : **DE-C- 945 147**

(73) Patentinhaber : **Rütgerswerke Aktiengesellschaft**
**Mainzer Landstrasse 217**
**D-6000 Frankfurt a.Main 1 (DE)**

(72) Erfinder : **Orth, Winfried, Dr.**
**Am Schachtelgraben 28**
**D-6735 Hassloch/Pfalz (DE)**
Erfinder : **Pastorek, Emmerich, Dr.**
**Grünbergerstrasse 90**
**D-6944 Hemsbach (DE)**
Erfinder : **Fickert, Werner, Dr.**
**Stockacher Strasse 14**
**D-6800 Mannheim (DE)**

**EP 0 082 542 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues, verbessertes Verfahren zur Herstellung von Pyridin-2,3-dicarbonsäure. Diese Verbindung wird allgemein als Chinolinsäure bezeichnet.

Chinolinsäure wird zur Herstellung pharmazeutisch wirksamer Verbindungen verwendet, wie z. B. Lokalanästhetika, Bakteriziden oder Verbindungen, die gegen Stoffwechselstörungen eingesetzt werden können.

Aus der Literatur sind verschiedene Verfahren zur Herstellung von Chinolinsäure bekannt. Sie beruhen teils auf der Oxidation von Chinolin und teils auf der Oxidation von 8-Hydroxychinolin.

Die zuerst von Hoogewerff und van Dorp (Ber. dtsch. chem. Ges. *12*, 747, (1879)) beschriebene Methode der Oxidation von Chinolin und Kaliumpermanganat in alkalischem Milieu erbringt nur mäßige Ausbeuten an Chinolinsäure neben einer großen Menge an anderen Reaktionsprodukten.

Die weiteren Verfahren zur Oxidation von Chinolin beruhen im wesentlichen auf der von Stix und Bulgatsch (Chem. Ber. *65*, 11 (1932)) beschriebenen Methode der Oxidation mit Wasserstoffperoxid in Anwesenheit eines Kupfersalzes. Da diese Reaktion recht schwierig zu handhaben ist, wurden in der Folgezeit Modifizierungen entwickelt, die eine bessere Steuerung der Reaktion und eine leichte Erhöhung der Ausbeute bedingen. Derartige Änderungen sind z. B. in der EP-A-0 024 197 oder EP-A-0 034 943 offenbart. Jedoch ist auch die nach den dort beschriebenen Verfahren erzielte Ausbeute an Chinolinsäure trotz extrem hohem Oxidationsmittelüberschuß nicht befriedigend. Außerdem entstehen bei all diesen Verfahren zuerst Kupfersalze der Chinolinsäure, aus denen die freie Säure mit Hilfe eines Sulfids freigesetzt werden muß. Abgesehen davon, daß dies ein weiterer, unerwünschter Reaktionsschritt ist, zeigt es sich, daß die vollständige Abtrennung der Kupferionen äußerst schwierig ist, so daß die entsprechend hergestellten Chinolinsäuren immer Spuren von Kupfer enthalten.

Aus Chem. Ber. *80*, 505 (1947) (C. A. *42*, 8801c (1948)) ist ein Verfahren bekannt, 8-Hydroxychinolin mit 67 %iger Salpetersäure zu Chinolinsäure zu oxidieren. Entgegen der Angaben dieser Literaturstelle wird bei diesem Verfahren zwar eine Vielzahl von Nebenprodukten, aber nur sehr wenig Chinolinsäure erhalten. Außerdem entstehen bei dieser Umsetzung große Mengen nitroser Gase.

In 68,7 %iger Ausbeute wird nach I. B. Chekmareva et al (C. A. 62, 16186a (1965)) Chinolinsäure durch Oxidation von 8-Hydroxychinolin mit Wasserstoffperoxid und Ozon erhalten. Das Verfahren ist jedoch so aufwendig, daß es lediglich von akademischem Interesse ist.

Die DE-PS 945 147 beschreibt die Oxidation von 8-Hydroxychinolin-5-sulfonsäure mit Natriumchlorit in Anwesenheit von Ammoniumvanadat zu Chinolinsäure. Erreicht wird eine Ausbeute von 72 %. Allerdings stellt die Synthese der Sulfonsäure einen zusätzlichen, unerwünschten Reaktionsschritt dar. Dieser Reaktionsschritt ist aber notwendig, weil sich 8-Hydroxychinolin mit Natriumchlorit nicht zu Chinolinsäure oxidieren läßt.

Es bestand daher die Aufgabe, ein neues, einfaches und umweltfreundliches Verfahren zu finden, mit dem sich in guter Ausbeute und hoher Reinheit Chinolinsäure herstellen läßt. Die Lösung der Aufgabe erfolgt durch das in den Ansprüchen 1 und 2 beschriebene Verfahren.

Als für das erfindungsgemäße Verfahren geeignete Chinolinderivate sind alle solche Chinoline, in denen am Benzolkern mindestens ein Wasserstoffatom durch eine aktivierende Gruppe substituiert ist. Aktivierende Gruppen im Sinne der Erfindung sind Substituenten —OR, —SR, —NRR′, $[—N^{\oplus}(R)_3]^+$, —NH—NRR′ oder NHCOR, wobei R und R′ gleich oder verschieden sein können und Wasserstoff oder substituierte oder unsubstituierte Alkyl-, Aralkyl- oder Cycloalkylreste mit 1-8 C-Atomen bedeuten.

Es ist für die Durchführung des Verfahrens nicht entscheidend, ob und gegebenenfalls mit welchen Gruppen noch weitere H-Atome am Benzolkern substituiert sind. So können sich wahlweise zusätzliche aktivierende Gruppen und/oder nicht aktivierend wirkende Substituenten am Benzolring befinden. Chinolinderivate im Sinne dieser Definition sind z. B. : 5-Äthoxy-, 6-Äthoxy-, 7-Äthoxy-, 8-Äthoxy-, 6-Äthoxy-7-brom-, 8-Äthoxy-6-nitro-, 5-Brom-8-methoxy-, 7-Brom-6-methoxy-, 5-Brom-8-propoxy-, 5-[2-Diäthylamino-äthoxy]-, 6-[2-Diäthylamino-äthoxy]-8-nitro-, 5-[2-Piperidino-äthoxy]-, 5-Methoxy-, 6-Methoxy-, 7-Methoxy-, 8-Methoxy-, 6-Methoxy-5,7-dimethyl-8-nitro-, 5,7-Dichlor-8-methoxy-, 5-Mercapto-, 6-Mercapto-, 7-Mercapto-, 8-Mercapto-, 6-Methylmercapto-, 6-Butylmercapto-, 6-Benzylmercapto-, 8-sec.-Butylmercapto-, 5,8-Dimercapto-, 6-Äthoxy-8-mercapto-, 5,7-Dichlor-8-mercapto-, 8-Methyl-5-mercapto-, 7-Methyl-8-mercapto-, 6-Methyl-8-mercapto-, 6-Methoxy-8-mercapto-, 5-Nitro-8-mercapto-, 5-Hydroxy-, 6-Hydroxy-, 7-Hydroxy-, 8-Hydroxy-, 5-Methyl-8-hydroxy-, 6-Methyl-8-hydroxy-, 5-Äthyl-8-hydroxy-, 7-Brom-8-hydroxy-, 5-Chlor-8-hydroxy-, 5,7-Dichlor-8-hydroxy-, 5-Octyl-8-hydroxy-, 5-Nitro-8-hydroxy-, 7-Nitro-8-hydroxy-, 5-Amino-, 6-Amino-, 7-Amino-, 8-Amino-, 5,8-Diamino-, 6,8-Diamino-, 8-Benzylamino-, 6-Methoxy-8- (4-aminopentyl) amino-, 5-Nitro-7,8-diamino-, 5,7-Dinitro-8-amino-, 8-Amino-6-chlor-, 6-Methoxy-8-amino-, 8-Acetylamino-6-äthoxy-, 6-Acetylamino-8-brom-, 8-Äthylamino-, 8-Benzylamino-6-methoxy-, 8-Butylamino-, 8-Cyclohexylamino-, 6-Diäthylamino-, 8-Diäthanolamino-, 8-Piperidino-, 6,8-Bisacetylamino-, 8-[4-Methylamino-piperidino]-, 5-Hydrazino-, 6-Hydrazino-, 7-Hydrazino-, 8-Hydrazino-, 8-Methyl-5-sulfonilamino-, 8-[2-Piperidino-äthoxy]-, 5,7,8-Triamino-chinolin, 8-Mercaptochinolin-5-sulfonsure, 8-Hydroxychinolin-5-sulfonsäure, 5-Aminochinolin-8-sulfonsäure, 5-Aminochinolin-6-carbonsäure.

Es wurde gefunden, daß diese in der beschriebenen Weise substituierten Chinolinderivate direkt zu Chinolinsäure oxidiert werden können, wenn man Chlorationen als Oxidationsmittel unter Verwendung

katalytischer Mengen von Vanadyl (V)-Kationen einsetzt. Dieses Ergebnis ist insofern überraschend, als Chlorate allgemein als die Oxidationsmittel gelten, mit denen man in den wenigsten Fällen eine gesteuerte partielle Oxidation durchführen kann und als das Redoxpotential der Chlorate (1,45 V) geringer ist als das der Chlorite (1,56 V).

Es ist dabei von Vorteil, daß bei diesem Oxidationsverfahren sowohl einfach substituierte Chinoline, die gezielt dazu synthetisiert werden können, ebenso oxidiert werden wie kompliziert aufgebaute Chinolinderivate, die unter Umständen bei anderen Synthesen als Abfallprodukte anfallen. Dazu kommt, daß im erfindungsgemäßen Verfahren auch beliebige Gemische von Chinolinderivaten zu Chinolinsäure oxidiert werden, solange jedes der eingesetzten Chinolinderivate am Benzolkern mindestens eine aktivierende Gruppe trägt. Dies bedeutet auch, daß an die Reinheit der Chinolinderivate insofern keine Anforaerungen gestellt werden, als auch Isomerengemische, die am Benzolkern in unterschiedlicher Position substituiert sind, im Verfahren eingesetzt werden können.

Vorteilhaft ist weiterhin sowohl die einfache Verfahrensdurchführung in wäßrigem Milieu, bei der keine toxischen Reaktionsprodukte anfallen, als auch die Tatsache, daß Chlorate billige Oxidationsmittel mit einem hohen Oxidationsvermögen sind. Es ist ein weiterer Vorteil des Verfahrens, daß man mit den stöchiometrisch notwendigen Mengen an Chlorat eine gute Ausbeute erzielt und in der Praxis lediglich zum schnelleren Beenden der Oxidationsreaktion einen geringen Überschuß an Oxidationsmittel verwendet.

Als Quelle für die Chlorationen können alle wasserlöslichen Chlorate eingesetzt werden. Vorzugsweise werden jedoch die Chlorate der Erdalkali- und Alkalimetalle und des Ammoniums verwendet. Insbesonders eignet sich das gut handhabbare, kristallwasserfreie, aber gut wasserlösliche Natriumchlorat.

Als Katalysator für diese Oxidationsreaktion dienen Vanadyl (V)-Kationen. Sie werden gebildet durch Auflösen von $V_2O_5$ oder wasserlöslicher Vanadate im sauren Reaktionsgemisch. Die Menge an Katalysator, die pro Reaktionsansatz eingesetzt wird, liegt zwischen 0,01 und 0,1 g $V_2O_5$ pro Mol an substituiertem Chinolinderivat, wobei naturgemäß eine höhere Katalysatormenge zu einer größeren Reaktionsgeschwindigkeit führt.

Die Reaktion wird in saurem Medium beim Siedepunkt des Reaktionsgemisches durchgeführt, wobei ein entsprechend substituiertes Chinolinderivat oder ein Gemisch mehrerer substituierter Chinolinderivate, Säure und Katalysator in wäßriger Lösung vorgelegt und erwärmt werden. Danach wird unter Rühren das Oxidationsmittel portionsweise zugegeben. Dabei kann festes Chlorat zudosiert werden, das sich im Reaktionsgemisch löst. Bevorzugt aber wird das Chlorat vorher in Wasser gelöst. Da die Oxidationsreaktion exotherm ist, ist nach Anspringen der Reaktion keine weitere Wärmezufuhr notwendig. Der Reaktionsverlauf wird lediglich durch die Zugabe des Chlorats gesteuert. Gegen Ende der Oxidationsreaktion bilden sich geringe Mengen gelbgrüner Gase, die gemeinsam mit dem bei der Reaktion entstehenden Kohlendioxid entweichen. Es ist daher zweckmäßig, das entweichende Gas durch eine Wäsche mit z. B. einer alkalischen Natriumsulfitlösung zu reinigen. Nach beendeter Reaktion wird das erhaltene Reaktionsgemisch leicht abgekühlt und der pH-Wert mit Hilfe einer Lauge auf pH 4-4,5 eingestellt. In diesem pH-Bereich liegt die Chinolinsäure als Betain gelöst vor, während durch Nebenreaktion entstandene Verunreinigungen ungelöst bleiben und, gegebenenfalls nach Adsorption an Aktivkohle, abgetrennt werden können. Das Filtrat wird angesäuert und die ausfallende Chinolinsäure abgetrennt und gewaschen. Sie wird dabei als ein fast weißes, reines Produkt in einer Ausbeute von etwa 73 Gew.-% der Theorie erhalten. Die abgetrennte Mutterlauge enthält noch weitere geringe Mengen an Chinolinsäure, die mit Hilfe eines Kupfersalzes abgetrennt und nach bekannten Verfahren isoliert werden können. Auf diese Weise können weitere 5 Gew.-% Chinolinsäure, allerdings mit Spuren von Kupfer verunreinigt, erhalten werden.

## Beispiel

871 g (6 Mol) 8-Hydroxychinolin werden in einer Lösung, bestehend aus 2 100 ml Wasser und 360 ml Schwefelsäure, 94 %ig, gelöst. Dabei steigt die Temperatur auf etwa 60 °C.

Danach werden 0,3 g Ammoniumvanadat als Katalysator zugegeben und die Lösung auf 85-90 °C erhitzt.

Unter intensivem Rühren werden innerhalb von 2-3 Stunden 1 788 g (16,8 Mol) Natriumchlorat, das in 2 400 ml Wasser gelöst wurde, so zugeführt, daß das Reaktionsgemisch bei gelindem Sieden (100-103 °C) ohne Wärmezufuhr gehalten werden kann. Am Anfang der Natriumchloratzugabe steigt die Reaktionstemperatur sehr schnell von 90 auf 103 °C. Nach Zugabe von ca. 2/3 des Oxidationsmittels tritt ein leichtes Schäumen auf. Bei Zugabe der letzten 5 % der benötigten Natriumchlorat-Menge entweichen geringe Mengen gelbgrüner Gase, die durch das aus der Reaktion entstandene Kohlendioxid stark verdünnt sind. Das Gas wird in drei nacheinander aufgebauten Absorptionsgefäßen aufgefangen. Die Gefäße sind mit wäßriger Natriumsulfitlösung gefüllt, unter Zugabe von Magnesiumoxid.

Nach der Natriumchloratzugabe wird die Lösung innerhalb von 20 Minuten auf etwa 90 °C abgekühlt und mit 961 ml Natronlauge, 50 %ig, auf pH 4-4,5 gebracht. Die Temperatur sollte dabei durch die Zutropfgeschwindigkeit bei etwa 100 °C gehalten werden.

Danach werden bei etwa 90 °C 60 g Aktivkohle, die vorher mit 150 ml Wasser vermischt wurden,

zugegeben. Dieses Gemisch wird 30 Min. gerührt, bei etwa 60 °C abgenutscht und zweimal mit je 200 ml 50 °C warmem Wasser nachgewaschen.

Das erhaltene Filtrat wird unter Rühren bei etwa 50 °C mit 800 ml Salzsäure, konzentriert, auf pH 1,5-1 gebracht und auf 20 °C abgekühlt. Das ausgefallene Produkt wird abgenutscht und viermal mit je 500 ml etwa 15 °C warmem Wasser, das vorher mit Salzsäure auf pH 1,5 gebracht wurde, nachgewaschen. Nach dem Trocknen bei 60-80 °C werden 730 g fast weiße Chinolinsäure erhalten.

Das entspricht einer Ausbeute von 72,8 % der Theorie.

## Patentansprüche

1. Verfahren zur Herstellung von Chinolinsäure durch katalytische Oxidation von substituierten Chinolinderivaten in saurem, wäßrigem Milieu, dadurch gekennzeichnet, daß Chinolinderivate der allgemeinen Formel

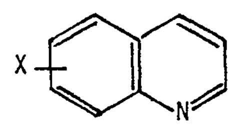

wobei X —OR, —SR, —NRR', $[-N^{\oplus}(R)_3]^+$, —NH—NRR' oder NHCOR bedeutet, und R und R' gleich oder verschieden sein können und Wasserstoff oder substituierte oder unsubstituierte Alkyl-, Aralkyl- oder Cycloalkylreste mit 1 bis 8 C-Atomen bedeuten und die restlichen Wasserstoffatome am Benzolkern nicht oder beliebig substituiert sind, mit Chlorationen in Gegenwart von Vanadyl (V)-Kationen als Katalysator im Temperaturbereich von 85 bis 103 °C oxidiert werden.

2. Verfahren nach Anspruch 1, dadurch gekennzeichnet, daß der pH-Wert des Reaktionsgemisches nach beendeter Reaktion auf 4-4,5 eingestellt und das Reaktionsgemisch filtriert und danach bis pH 1-1,5 angesäuert und abgekühlt werden.

## Claims

1. A process for the preparation of quinolinic acid by the catalytic oxidation of substituted quinoline derivates in an acidic, aqueous medium, characterised in that quinoline derivates having the general formula

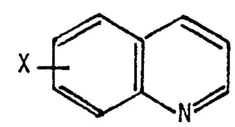

in which X stands for —OR, —SR, —NRR', $[-N^+(R)_3]^+$, —NH—NRR' or —NHCOR and R and R' may be the same or different and may represent hydrogen, or a substituted or an unsubstituted alkyl, aralkyl or cycloalkyl group of 1 to 8 carbon atoms and in which the remaining hydrogen atoms on the benzene nucleus may or may not be substituted, said oxidation being carried out in the temperature range of 85 to 103 °C using a chlorate oxidizing agent in the presence of vanadyl-V-cations as the catalyst.

2. A process of claim 1, characterised in that the pH-value of the reaction mixture is adjusted to 4-4,5 after the reaction is finished and the reaction mixture is filtered and then acidified to a pH of 1-1,5 and cooled off.

## Revendications

1. Procédé de préparation d'acide quinoléique par oxydation catalytique de dérivés quinoléiques substitués en milieu acide aqueux, caractérisé par le fait que des dérivés de quinoléine de formule générale

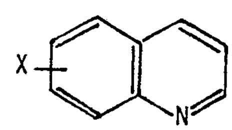

dans laquelle X signifie —OR, —SR, —NRR', $[-N^{\oplus}(R)_3]^+$, —NH—NRR' ou NHCOR, R et R' étant identiques ou différents l'un de l'autre et représentant de l'hydrogène ou des radicaux alcoyle, aralcoyle ou cycloalcoyle substitués ou non substitués avec 1 à 8 atomes de carbone, les atomes d'hydrogène

restants du noyau benzénique n'étant pas substitués ou étant substitués à volonté, sont oxydés à l'aide d'ions chlorate en présence de cations vanadyl (V) à titre de catalyseurs, dans le domaine de températures de 85 à 103 °C.

2. Procédé selon la revendication 1, caractérisé par le fait que la valeur du pH du mélange réactionnel est réglée à 4-4,5 une fois la réaction terminée et que le mélange réactionnel est filtré puis acidifié jusqu'à pH 1-1,5 et refroidi.